# EUROPEAN PATENT SPECIFICATION

(11) **EP 1 809 234 B1**
(45) Date of publication and mention of the grant of the patent: **01.02.2012**
(21) Application number: 05857846.9
(22) Date of filing: 19.10.2005
(51) Int. Cl.: A61K 8/03, A61K 8/04, A61Q 5/06, A61K 8/34, A61K 8/60

(54) **CLEAR, TOW-PHASE, FOAM-FORMING AEROSOL HAIRSTYLING PRODUCT**
KLARES, SCHAUMFORMENDES ZWEIPHASEN-SPRAYPRODUKT FÜR DAS HAAR
PRODUIT DE COIFFURE TRANSPARENT, BIPHASIQUE ET MOUSSANT EN AEROSOL

(30) Priority: 10.11.2004 DE 102004054278
(43) Date of publication of application: 25.07.2007
(73) Proprietor: Wella GmbH, 65825 Schwalbach am Taunus (DE)
(72) Inventor: FRANZKE, Michael, 64380 Rossdorf (DE); MOENKS, Monika, CH-3185 Schmitten (CH); SCHIEMANN, Hartmut, 36088 Hünfeld (DE); FLORIG, Ellen, 64689 Grasellenbach (DE); BAECKER, Sabine, 65428 Rüsselsheim (DE); ROETTGER, Cornelia, 37632 Eschershausen (DE); GAENGER, Klaus, 64319 Pfungstadt (DE)
(86) International application number: PCT/EP2005/011215
(87) International publication number: WO 2006/050788

(56) References cited:
- EP-A- 0 250 181
- EP-A- 1 169 998
- WO-A-98/36041
- DE-A1- 19 915 837
- US-A- 3 219 656
- US-A- 6 113 888
- US-A1- 2002 197 213

## Description

The object of the invention is a hair care product consisting of transparent, pressure-resistant aerosol packaging, a device for foaming a composition contained in the aerosol packaging, and a foaming composition of at least two clear liquid phases separated from each other. The composition contains water; water-soluble liquid alcohols; hair-conditioning, hair-setting, or film-forming polymers; hair-conditioning cationic surfactants; foam-forming or foam-stabilizing, nonionic or non-cationic ionogenic surfactants; as well as water-insoluble, liquified aerosol propellants.

Hair treatment agents that are released as a foam from pressurized gas packaging are known and are also characterized as aerosol foams, foam aerosols, styling foams, conditioning foams, or mousse. The products primarily consist of pressurized packaging, a foam head, a liquid, aqueous active ingredient phase, and a liquified propellant phase. Normally, these products require opaque packaging, for example, made of metal or opaque plastic because, at the interface between the liquified propellant phase and the active ingredient phase, a non-homogenous, non-cosmetic-like, and unattractive-looking, turbid, smear-forming substance forms due to interactions from the propellant, active ingredients, and the additives, and/or there can be turbidity from one or both phases due to the synergy of the commonly used ingredients. Transparent packaging is desirable, however, due to the simple visual recognition of the fill level, the simple visual recognition of sufficient phase mixing after shaking before use, the recognition of a color design in the ingredients if applicable, as well as the fact that transparent products are generally more attractive.

Clear, two-phase, foam-forming aerosol hair-care products are known from EP 1 169 998 A2. The products have two clear liquid phases that are distinctly separate from one another. A particularly distinct phase separation, which can even be reestablished after shaking, is obtained by adding a water-soluble salt to the hydrophilic phase and by adding a water-insoluble hydrophobic substance, which is soluble in the liquified propellant, with said substance being oil and fat in particular. The addition of salts as well as oils and fats that is necessary for the phase separation can cause undesirable influences. Thus, the additives can have a "heavying" effect, particularly when applied to fine hair, which also makes the manufacture of volume products for fine hair difficult. The additives can also sometimes be softeners for the hair setting polymers, which impedes the manufacture of styling products with strong holds. Omitting the additives, however, reduces the quality of phase separation to an undesirable level.

Thus, the task arose of how to provide a transparent hair product that could exist under pressure to form foam; the product would also need to have phase separation between the liquified propellant phase and the water-containing active ingredient phase that is reversible after shaking and recognizable from the outside; the product would also need to enable the production of volume and styling products with satisfactory volume and setting effects and also be producible with absolutely no or a very small amount of salts and hydrophobic fats or oils.

The object of the present invention is a hair care product consisting of transparent, pressure-resistant aerosol packaging, a device for foaming a composition contained in the aerosol packaging and a foamable composition of at least two clear liquid phases separated from each other, wherein the composition contains
(A) water;
(B) at least 15 wt%, based on the composition without propellant, of a water-soluble liquid alcohol;
(C) at least one polymer selected from the hair-conditioning polymers, the hair-setting polymers, and the film-forming polymers;
(D) at least one hair-conditioning cationic surfactant;
(E) at least one foam-forming or foam-stabilizing surfactant, selected from the group consisting of nonionic surfactants, with an HLB value of at least 10 and zwitterionic surfactants;
(F) at least one water-insoluble propellant that is liquified under the pressure conditions in the aerosol packaging.

The compositions according to the present invention distinguish themselves by an increased alcohol content of at least 15 wt%. Although the quality of the phase separation can be improved with increasing alcohol content, the foam quality normally is worsened because the alcohols act as foam inhibitors. Good phase separation as well as good foam quality can be obtained by the simultaneous choice of the alcohol quantity and suitable selection of the surfactants (E) according to the present invention without influencing the volume or styling effects of the product to any significant extent.

Both liquid phases are clear in the sense of the invention if no turbidity or smearing can be seen with the naked eye. The phases are liquid in the sense of the-invention if they are flowable. This applies both to liquids when viewed from a more physical perspective as well as fluid gels that automatically flow downwards when placed at an angle (45°) at 20 °C (68 °F). Both phases are separated from each other in the sense of the invention if they only form one single, horizontal interface. After shaking, this interface completely reestablishes itself preferably within 24 hours or less. It is preferred if the phases are distinctly separate from each other, i.e. no border layer but instead only a separating line can be seen between the phases or the separating line between the phase is less than 1 mm (0.039 in) and preferably less than 0.1 mm (0.0039 in). Foamable compositions are those that form stable foam for at least a short period (e.g. 30 seconds at a minimum) using suitable devices for foaming such as aerosol packaging with a foam head.

### Hydrophilic phase solvent

Water and water-soluble alcohols are the primary solvents. The water content is preferably from 50 to 75 wt%, with 55 to 75 wt% being especially preferred, based on the composition without aerosol propellant. The alcohols are liquid and water-soluble at room temperature (25 °C (77 °F)), i.e. it is preferable if at least 20 g (0.71 oz) or at least 50 g (1.76 oz) are soluble in 100 ml (3.38 oz); unlimited solubility is particularly preferred. The alcohols used can be those lower monovalent alcohols with 1 to 4 C atoms that are customarily used for cosmetic purposes such as ethanol and isopropanol or polyvalent alcohols with 2 to 5 C atoms such as ethylene glycol, glycerin, propylene glycol, butylene glycol, or pentanediol. Ethanol, either as the sole alcohol or in a mixture with ethylene glycol, glycerin, or propylene glycol, is particularly preferred. The alcohol content is at least 15 wt%, with 20 to 35 wt% being preferred, and 20 to 30 wt% being particularly preferred, each instance based on the composition without aerosol propellant. The hydrophilic phase is preferably in a pH range of from 2 to 8, with a pH of from 4 to 7 being particularly preferred. In the event that a zwitterionic surfactant is used as the foam-forming or foam-stabilizing surfactant, then the pH value is preferably greater than 3.5, and particularly greater than 4.0. The foam quality can be impaired with a low pH value of about 3 due to the cationic character of the zwitterionic surfactant, which can then be too strong. An acid range can be set with a cosmetically compatible organic or inorganic acid such as formic acid, tartaric acid, malic acid, maleic acid, fumaric acid, pyrrolidone carboxylic acid, citric acid, lactic acid, sulfuric acid, acetic acid, hydrochloric acid, and phosphoric acid among others. A basic pH range can be set with suitable organic or inorganic bases, e.g. amino alcohols such as aminomethylpropanol (AMP), triethanolamine, monoethanolamine, or tetrahydroxypropyl ethylenediamine as well as ammonia, NaOH, KOH, and others.

### Polymers

The composition of the product according to the invention contains at least one hair-setting and/or hair-conditioning and/or film-forming polymer. The polymers are preferably present in a quantity of from 0.01 to 20 wt% or from 0.5 to 10 wt%, with a wt% of from 1 to 8 being particularly preferred. These polymers can be anionic polymers, in other words, polymers with anionic or anionizable groups, or cationic polymers, in other words, polymers with cationic or cationizable groups, or zwitterionic polymers, in other words, polymers with cationic and anionic groups, or amphoteric polymers, in other words, polymers with acidic and basic groups, or nonionic polymers. Anionizable groups are understood to be acid groups such as, for example, carboxylic acid, sulfonic acid, or phosphoric acid groups, which can be deprotonated by means of conventional bases such as, for example, organic amines or alkaline or alkaline earth hydroxides. Groups that can be cationized are basic groups such as primary, secondary, or tertiary amine groups that can be protonated using typical acids. Those polymers that are sufficiently soluble in water/alcohol mixtures are preferred in order to provide the completely dissolved form in the hydrophilic phase according to the present invention.

Film-forming polymers are, in particular, those polymers listed in the International Cosmetic Ingredient Dictionary and Handbook, 9^{th} edition, with the function of "Film Formers" and/or polymers that are capable of leaving a polymer film on the hair when used in a 0.01 to 5% aqueous, alcohol, or aqueous alcohol solution. Hair-setting polymers are, in particular, those polymers listed in the International Cosmetic Ingredient Dictionary and Handbook, 9^{th} edition, with the function of "Hair Fixatives." Hair-conditioning polymers are, in particular, those materials listed in the International Cosmetic Ingredient Dictionary and Handbook, 9^{th} edition, with the function of "Hair Conditioning Agents," provided that polymers are meant. Examples of polymers in the sense of the invention are listed below.

Anionic polymers can be partially or completely neutralized with a basic neutralizing agent. A preferred degree of neutralization is from 50 to 100 %, and 70-100% is especially preferred. An organic or inorganic base can be used as the neutralizing agent. Particular examples of bases are amino alkanols such as, for example, aminomethylpropanol (AMP), triethanolamine or monoethanolamine, and also ammonia, NaOH, and KOH among others.

The anionic polymer can be a homo- or copolymer with acid group-containing monomer units derived from natural or synthetic sources, which, if necessary, can be polymerized with comonomers that contain no acid groups. Among the acid groups that can be considered are sulfonic acid, phosphoric acid and carboxylic acid groups, of which the carboxylic acid groups are preferred. Suitable acid group-containing monomers are, for example, acrylic acid, methacrylic acid, crotonic acid, maleic acid, and maleic anhydride, maleic acid monoesters, especially the C-1 to C-7 alkyl monoesters of maleic acid, as well as aldehydocarboxylic acids or ketocarboxylic acids. Comonomers that are not substituted with acid groups are, for example, acrylamide, methacrylamide, alkyl- and dialkylacrylamide, alkyl- and dialkylmethacrylamide, alkyl acrylate, alkyl methacrylate, vinylcaprolactone, vinylpyrrolidone, vinyl ester, vinyl alcohol, propylene glycol or ethylene glycol, amine-substituted vinyl monomers such as, for example, dialkylaminoalkyl acrylate, dialkylaminoalkyl methacrylate, monoalkylaminoalkyl acrylate and monoalkylaminoalkyl methacrylate, wherein the alkyl groups of these monomers are preferably C-1 to C-7 alkyl groups, with C-1 to C-3 alkyl groups being especially preferred.

Suitable polymers with acid groups are especially homopolymers of acrylic acid or methacrylic acid, copolymers of acrylic acid or methacrylic acid with monomers selected from acrylic acid or methacrylic acid esters, acrylamides, methacrylamides and vinylpyrrolidone, homopolymers of crotonic acid as well as copolymers of crotonic acid with monomers selected from vinyl esters, acrylic acid or methacrylic acid esters, acrylamides and methacrylamides that are uncrosslinked or crosslinked with polyfunctional agents. A suitable natural polymer is, for example, shellac.

Preferred polymers with acid groups are:
Terpolymers from acrylic acid, alkyl acrylate, and N-alkylacrylamide (INCI designation: Acrylate/Acrylamide Copolymer), especially terpolymers from acrylic acid, ethyl acrylate and N-tert-butylacrylamide; crosslinked or uncrosslinked vinyl acetate/crotonic acid copolymers (INCI designation: VA/Crotonate Copolymer); copolymers from one or more C-1 to C-5 alkyl acrylates, especially C-2 to C-4 alkyl acrylates and at least one monomer selected from acrylic acid or methacrylic acid (INCI designation: Acrylate Copolymer), e.g. terpolymers from tert-butyl acrylate, ethyl acrylate and methacrylic acid; sodium polystyrenesulfonate; vinylacetate/crotonic acid/vinyl alkanoate copolymers, for example, copolymers from vinyl acetate, crotonic acid and vinyl propionate; copolymers from vinyl acetate, crotonic acid and vinyl neodecanoate (INCI designations: VA/Crotonate/Vinyl Propionate Copolymer, VA/Crotonate/Vinyl Neodecanoate Copolymer); aminomethylpropanol acrylate copolymers; copolymers from vinylpyrrolidone and at least one further monomer selected from acrylic acid and methacrylic acid as well as, if necessary, acrylic acid esters and methacrylic acid esters; copolymers from methyl vinyl ether and maleic acid monoalkylesters (INCI designations: Ethyl Ester of PVM/MA Copolymer, Butyl Ester of PVM/MA Copolymer); aminomethylpropanol salts of copolymers from allyl methacrylate and at least one further monomer selected from acrylic acid, and methacrylic acid as well as, if necessary, acrylic acid esters and methacrylic acid esters; crosslinked copolymers from ethyl acrylate and methacrylic acid; copolymers from vinyl acetate, mono-n-butyl maleate and isobornyl acrylate; copolymers from two or more monomers selected from acrylic acid and methacrylic acid as well as, if necessary, acrylic acid esters and methacrylic acid esters; copolymers from octylacrylamide and at least one monomer selected from acrylic acid and methacrylic acid as well as, if necessary, acrylic acid esters and methacrylic acid esters; polyesters from diglycol, cyclohexanedimethanol, isophthalic acid and sulfoisophthalic acid, wherein the alkyl groups of the aforementioned polymers as a rule preferably possess 1, 2, 3, or 4 C atoms.

Preferred zwitterionic or amphoteric polymers are:
Copolymers formed from alkylacrylamide, alkylaminoalkyl methacrylate, and two or more monomers from acrylic acid and methacrylic acid as well as, if necessary, their esters, especially copolymers from octylacrylamide, acrylic acid, butylaminoethyl methacrylate, methyl methacrylate and hydroxypropyl methacrylate (INCI designation: Octylacrylamide/AcrylateButylaminoethyl Methacrylate Copolymer); copolymers, that are formed from at least one of a first type of monomer that possesses quaternary amino groups and at least one of a second type of monomer that possesses acid groups; copolymers from fatty alcohol acrylates, alkylamine oxide methacrylate and at least one monomer selected from acrylic acid and methacrylic acid as well as, if necessary, acrylic acid esters and methacrylic acid esters, especially copolymers from lauryl acrylate, stearyl acrylate, ethylamine oxide methacrylate and at least one monomer selected from acrylic acid and methacrylic acid as well as, if necessary, their esters; copolymers from methacryloyl ethyl betaine and at least one monomer selected from methacrylic acid and methacrylic acid esters; copolymers from acrylic acid, methyl acrylate and methacrylamidopropyltrimethylammonium chloride (INCI designation: Polyquatemium-47); copolymers from acrylamidopropyltrimethylammonium chloride and acrylates or copolymers from acrylamide, acrylamidopropyltrimethylammonium chloride, 2-amidopropylacrylamide sulfonate and dimethylaminopropylamine (INCI designation: Polyquaternium-43); oligomers or polymers, producible from quaternary crotonoylbetaines or quaternary crotonoylbetaine esters.

Cationic polymers are especially those with primary, secondary, tertiary or quaternary amino groups. The cationic charge density will be preferably from 1 to 7 meq/g. Suitable cationic polymers preferably contain quaternary amino groups. Cationic polymers can be homo- or copolymers, where the quaternary nitrogen groups are contained either in the polymer chain or preferably as substituents on one or more of the monomers. The ammonium group-containing monomers can be copolymerized with non-cationic monomers. Suitable cationic monomer are unsaturated compounds that can undergo radical polymerization, which bear at least one cationic group, especially ammonium-substituted vinyl monomers such as, for example, trialkylmethacryloxyalkylammonium, trialkylacryloxyalkylammonium, dialkyldiallylammonium and quaternary vinylammonium monomers with cyclic, cationic nitrogen-containing groups such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups such, as for example, C-1 to C-7 alkyl groups, and especially preferred are C-1 to C-3 alkyl groups.

The ammonium group-containing monomers can be copolymerized with non-cationic monomers. Suitable comonomers are, for example, acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, for example vinyl acetate, vinyl alcohol, propylene glycol or ethylene glycol, where the alkyl groups of these monomers are preferably C-1 to C-7 alkyl groups, and especially preferred are C-1 to C-3 alkyl groups.

Suitable polymers with quaternary amino groups are, for example, those described in the CTFA Cosmetic Ingredient Dictionary under the designations Polyquaternium such as methylvinylimidazolium chloride/vinylpyrrolidone copolymer (Polyquaternium-16) or quatemized vinylpyrrolidone/dimethylaminoethyl methacrylate copolymer (Polyquaternium-11) as well as quaternary silicone polymers or silicone oligomers such as, for example, silicone polymers with quaternary end groups (Quatemium-80).

Preferred cationic polymers of synthetic origin:
Poly(dimethyldiallylammonium chloride); copolymers from acrylamide and dimethyldiallylammonium chloride; quaternary ammonium polymers, formed by the reaction of diethyl sulfate with a copolymer from vinylpyrrolidone and dimethylaminoethyl methacrylate, especially vinylpyrrolidone/dimethylaminoethyl methacrylate methosulfate copolymer (e.g. Gafquat^{®} 755 N, Gafquat^{®} 734); quaternary ammonium polymers from methylvinylimidazolium chloride and vinylpyrrolidone (e.g. LUVIQUAT^{®} HM 550, LUVIQUAT^{®} FC 905); Polyquatemium-35; Polyquatemium-57; polymers from trimethylammonium ethyl methacrylate chloride; terpolymers from dimethyldiallylammonium chloride, sodium acrylate and acrylamide (e.g. Merquat^{®} Plus 3300); copolymers from vinylpyrrolidone, dimethylaminopropyl methacrylamide, and methacryloylaminopropyllauryldimethylammonium chloride; terpolymers from vinylpyrrolidone, dimethylaminoethyl methacrylate, and vinylcaprolactam (e.g. Gaffix^{®} VC 713); vinylpyrrolidone/methacrylamidopropyltrimethylammonium chloride copolymers (e.g. Gafquat^{®} HS 100); copolymers from vinylpyrrolidone and dimethylaminoethyl methacrylate; copolymers from vinylpyrrolidone, vinylcaprolactam, and dimethylaminopropylacrylamide; poly- or oligoesters formed from at least one first type of monomer that is selected from hydroxyacids substituted with at least one quaternary ammonium group; dimethylpolysiloxane substituted with quaternary ammonium groups in the terminal positions.

Suitable cationic polymers that are derived from natural polymers are, in particular, cationic derivatives of polysaccharides, for example, cationic cellulose derivatives, starch, or guar. Furthermore, chitosan and chitosan derivatives are suitable. Cationic polysaccharides are, for example, represented by the general formula

G-O-B-N⁺R^{a}R^{b}R^{c} X⁻

G is an anhydroglucose residue, for example, starch or cellulose anhydroglucoses;
B is a divalent bonding group, for example, alkylene, oxyalkylene, polyoxyalkylene or hydroxyalkylene;
R^{a}, R^{b} and R^{c} are, independently from one another, alkyl, aryl, alkylaryl, arylalkyl, alkoxyalkyl or alkoxyaryl, any of which can have up to 22 C atoms, wherein the total number of C atoms in R^{a}, R^{b}, and R^{c} is preferably a maximum of 20;
X is a conventional counter-anion, for example, a halogen, acetate, phosphate, nitrate, or alkylsulfate, preferably a chloride. Cationic celluloses are, for example, those with the INCI designations Polyquatemium-10 or Polyquaternium-24. A suitable cationic guar derivative has for example the INCI designation Guar Hydroxypropyltrimonium Chloride.

Cationic cellulose derivatives are those that have at least one quaternary ammonium group, e.g. a copolymer made of hydroxyethyl cellulose and diallyldimethyl ammonium chloride (Polyquaternium-4), or the reaction product made of hydroxyethyl cellulose and an epoxide substituted with a trialkyl ammonium group (Polyquaternium-10), wherein the alkyl groups can have 1 to 20 C atoms, and methyl groups are preferred. The molecular weight is preferably between 100,000 and 600,000, but 200,000 to 400,000 is especially preferred. The nitrogen content is preferably 0.5 to 4%, with 1.5 to 3% being especially preferred. The preferred cellulose derivative is Polyquaternium-4, which is sold under the trade names Celquat^{®} H100 and Celquat^{®} L200, of which Celquat^{®} L200 is especially preferred.

Especially preferred cationic polymers are chitosan, chitosan salts, and chitosan derivatives. Chitosans that can be used in the present invention can be fully or partially deacetylated chitins. By way of example, the molecular weight can be distributed over a broad range, from 20,000 to about 5 million g/mol (705 to about 176,369 oz/mol), e.g. from 30,000 to 70,000 g/mol (1,058 to 2,469 oz/mol). The molecular weight is, however, preferably more than 100,000 g/mol (3,527 oz/mol), with 200,000 to 700,000 g/mol (7,054 to 24,692 oz/mol) being especially preferred. The degree of deacetylation is preferably from 10 to 99%, and especially preferably from 60 to 99%. A preferred chitosan salt is chitosonium pyrrolidone carboxylate, e.g. Kytamer^{®} PC with a molecular weight of from about 200,000 to 300,000 g/mol (7,054 to 10,582 oz/mol) and a degree of deacetylation of from 70 to 85%. Chitosan derivatives that can be considered include quatemized, alkylated, or hydroxyalkylated derivatives, e.g. hydroxyethyl, hydroxypropyl, or hydroxybutyl chitosan. The chitosans or chitosan derivatives are preferably present in their neutralized or partially neutralized form. The degree of neutralization will be preferably at least 50%, especially preferably between 70 and 100%, as calculated on the basis of the number of free basic groups. For the neutralization agent, in principle any cosmetically compatible inorganic or organic acids can be used such as, for example, formic acid, tartaric acid, malic acid, lactic acid, citric acid, pyrrolidonecarboxylic acid, hydrochloric acid and others, of which pyrrolidonecarboxylic acid is especially preferred.

Preferred cationic polymers derived from natural sources:
Cationic cellulose derivatives from hydroxyethylcellulose and diallyldimethylammonium chloride; cationic cellulose derivatives from hydroxyethylcellulose and with a trimethylammonium-substituted epoxide; chitosan and its salts; hydroxyalkylchitosans and their salts; alkylhydroxyalkylchitosans and their salts; N-hydroxyalkylchitosan alkyl ethers; N-hydroxyalkylchitosan benzyl ether.

Suitable nonionic polymers are homo- or copolymers that are formed from at least one of the following monomers: vinylpyrrolidone, vinylcaprolactam, vinyl esters such as, for example, vinyl acetate, vinyl alcohol, acrylamide, methacrylamide, alkyl- and dialkylacrylamide, alkyl- and dialkylmethacrylamide, alkyl acrylate, alkyl methacrylate, propylene glycol or ethylene glycol, where the alkyl groups in these monomers are preferably C-1 to C-7 alkyl groups, and C-1 to C-3 alkyl groups are especially preferred. Suitable homopolymers are, for example, those of vinylcaprolactam, vinylpyrrolidone or N-vinylformamide. Further suitable synthetic, film-forming, nonionic, hair-setting polymers are, for example, copolymerides from vinylpyrrolidone and vinyl acetate, terpolymers from vinylpyrrolidone, vinyl acetate and vinyl propionate, polyacrylamides; polyvinyl alcohols as well as polyethylene glycol/polypropylene glycol copolymers. Suitable natural, film-forming polymers are, for example, cellulose derivatives, e.g. hydroxyalkylcellulose.

Preferred nonionic polymers are:
Polyvinylpyrrolidone, polyvinylcaprolactam, vinylpyrrolidone/vinyl acetate copolymers, polyvinyl alcohol, isobutylene/ethylmaleimide/hydroxyethylmaleimide copolymer; copolymers from vinylpyrrolidone, vinyl acetate and vinyl propionate.

Preferred polymer combinations are the following in particular:
■ Cationic cellulose derivatives from hydroxyethyl cellulose and diallyldimethylammonium chloride in conjunction with vinylpyrrolidone/vinyl acetate copolymers;
■ Chitosan in conjunction with polyvinylpyrrolidone;
■ Quaternary ammonium polymers from methylvinylimidazolium chloride and vinylpyrrolidone in conjunction with chitosan and/or vinylpyrrolidone/vinyl acetate copolymers, and/or polyvinylpyrrolidone.

### Cationic surfactants

The cationic surfactants (D) are contained in the composition of the product according to the present invention preferably in a quantity of from 0.01 to 10 or from 0.05 to 5, with 0.1 to 2.5 wt% being especially preferred, based on the composition without aerosol propellant. Suitable cationic surfactants are, in particular, surfactants that have a substantivity to human hair based on cationic groups or those that can be cationized, particularly protonated amine groups or quaternary ammonium groups.

Suitable cationic surfactants are, particularly, those of the general formula

N⁽⁺⁾R¹R²R³R⁴ X⁽⁻⁾ (I)

wherein R1 to R4, independently from one another, represent aliphatic groups, aromatic groups, alkoxy groups, polyoxyalkylene groups, alkylamido groups, hydroxyalkyl groups, aryl groups, or alkaryl groups with 1 to 22 C atoms, and wherein at least one of the residues R1 to R4 possesses at least 8 C atoms and X⁽⁻⁾ represents an anion, e.g. a halogen, acetate, phosphate, nitrate, or alkylsulfate, but preferably a chloride. The aliphatic groups can also contain cross linkages or other groups such as additional amino groups in addition to the carbon and hydrogen atoms.

Examples of suitable cationic surfactants are the chlorides or bromides of alkyl dimethyl benzyl ammonium salts, alkyl trimethyl ammonium salts, for example, cetyltrimethylammonium chloride or bromide, tetradecyltrimethyl ammonium chloride or bromide, alkyldimethyl hydroxyethyl ammonium chlorides or bromides, the dialkyldimethyl ammonium chlorides or bromides, alkylpyridinium salt, for example, lauryl or cetylpyridinium chloride, alkylamido ethyl trimethyl ammonium ether sulfates, as well as compounds with cationic character such as amine oxides, for example, alkyl methyl amine oxides or alkyl amino ethyl dimethyl amine oxides. Especially preferred is cetyltrimethylammonium chloride.

### Foam-forming and foam-stabilizing surfactants

The foam-forming and foam-stabilizing surfactants strengthen the foam capacity of the composition of the present invention, i.e. they increase the volume and/or the stability of the foam created when the product is released from the aerosol packaging, despite the high alcohol content. They are present in a quantity of preferably 0.1 to 5 wt%, with 0.5 to 3 wt% being preferred, based on the composition without aerosol propellant.

The surfactants (E) are selected from nonionic surfactants with an HLB value as classified by Griffin that is greater than or equal to 10, but is preferably in a range of from 11 to 15, as well as from zwitterionic surfactants. Preferred nonionic surfactants are alkylpolyglucosides (APG). These are acetal-linked glucosides that can be present as mono-, di-, or oligoglucosides or a mixture thereof. They can be manufactured as a homogenous mixture via an acidic catalyzed reaction (Fischer reaction) from glucose, starch, or n-butyl glucosides with fatty alcohols. This results in complex mixtures of alkyl monoglucoside, alkyl diglucosides, and alkyl oligoglucosides (particularly triosides and tetraosides) with e.g. 8 to 22, 8 to 20, or preferably 10 to 16 C atoms in the alkyl group. The average degree of polymerisation is normally between 1 and 3, e.g. between 1.2 and 1.5. The HLB values are normally above 10 and can vary between 11 and 15 due to variation in the hydrophobic group and/or the degree of polymerisation. APGs are known under the INCI designations Coco-Glucoside, Caprylyl/Capryl Glucoside, Cetearyl Glucoside, Decyl Glucoside, Lauryl Glucoside, and Myristyl Glucoside.

Zwitterionic surfactants are surfactants with at least one quaternary ammonium group and at least one anionic group, selected from carboxylate, sulfate, sulfonate, phosphate, and phosphonate groups, e.g. compounds of the general formula

R¹-N⁽⁺⁾(R²)₂-A-Z⁽⁻⁾

wherein R1 represents a linear or branched alkyl, alkenyl, or hydroxyalkyl group with from 8 to 22 C atoms and from 0 to 10 ethylene oxide units and from 0 to 1 glycerol units or a fatty acid amidoalkylene group with 8 to 22 C atoms in the fatty acid group and from 1 to 4, preferably 2 or 3, C atoms in the alkylene group; R2 is an alkyl, hydroxyalkyl, or carboxyalkyl group with from 1 to 4 C atoms; A is an alkylene or hydroxyalkylene group with from 1 to 4 C atoms and 0 or 1 hydroxy groups, and Z represents a carboxylate, sulfate, phosphonate, or phosphate group. Zwitterionic phosphate surfactants are, e.g. known under the INCI designation Sodium Lauroampho PG-Acetate Phosphate.

Zwitterionic surfactants with a betaine structure are preferred, e.g. those of the formula

R-N⁽⁺⁾(CH₃)₂-(CH₂)ₓ-CO₂⁽⁻⁾

wherein R means a fatty alkyl residue with from 8 to 22 C atoms or a fatty alkyl amido alkylene residue with from 8 to 22 C atoms in the fatty alkyl group and from 1 to 3 C atoms in the alkylene group, and wherein x means the numbers 1, 2, or 3. These types of surfactants are known under the INCI designations Cocamidopropyl Betaine, Cocamidoethyl Betaine, Lauramidopropyl Betaine, Stearamidopropyl Betaine, Cetyl Betaine, Lauryl Betaine, Oleyl Betaine, Stearyl Betaine, or Coco-Betaine.

Zwitterionic surfactants with a sultaine structure are also preferred, e.g. those of the formula

R-N⁽⁺⁾(CH₃)₂-A-SO₃⁽⁻⁾

wherein R means a fatty alkyl residue with from 8 to 22 C atoms or a fatty alkyl amido alkylene residue with from 8 to 22 C atoms in the fatty alkyl group and from 1 to 3 C atoms in the alkylene group, and wherein A means an alkylene or hydroxyalkylene group with 1, 2, or 3 C atoms. These types of surfactants are known under the INCI designations Cocamidoproyl Hydroxysultaine, Lauramidopropyl Hydroxysultaine, Coco-Sultaine, Lauryl Sultaine, and Lauryl Hydroxysultaine.

### Aerosol propellants

The aerosol propellants according to the present invention are hydrophobic materials that are gaseous under normal conditions, i.e. at normal pressure (1,013 mbar) and ambient temperature (20 °C (68 °F)), are present in liquified form when filled under pressure, and are at least partially insoluble in the hydrophilic phase and therefore form a second, hydrophobic liquid phase. Considered suitable are, particularly, C3 and C4 hydrocarbons such as propane, isobutane, n-butane, or mixtures thereof, as well as fluorohydrocarbons such as F 152 (1,1-difluoroethane) or F 134 (tetrafluoroethane). Mixtures with hydrophilic, partially water-soluble aerosol propellants, such as dimethylether, are also possible provided they are not used as the sole aerosol propellant because otherwise the second phase would not form. Pure dimethylether also has the disadvantage that it is not compatible with the especially preferred packaging materials made from transparent plastic, particularly polyethylene terephthalate. The container wall can become soft and unstable due to the dimethylether; materials can be extracted from the container wall, or the diffusion of ingredients from the active ingredient solution can go into or through the container wall. Especially preferred are mixtures of propane and butane, as well as mixtures of propane, butane, and dimethylether.

The quantity of aerosol propellants used is preferably selected so that, after filling, the pressure in the aerosol packaging is a maximum of 3 bar, with a maximum of 2.7 bar at 20 °C (68 °F) being especially preferred, and/or the fill ratio of propellant-free composition to aerosol propellant is in the range of from 80:20 to 97:3, with 85:15 to 95:5 being preferred.

The product according to the present invention is packaged in suitable, pressure-resistant aerosol packaging and has, as an additional component, a device for foaming the composition contained in the packaging, wherein said device enables the foaming of the composition when propellant is used. As a suitable foaming device, for example, a commercially available aerosol foaming head can be used. The packaging is made of transparent material, through which the consistency, the fill level, the color of the composition, and the degree of mixing or the separation of the two liquid phases are recognizable. Possible materials for the packaging are, for example, glass and transparent, pressure-resistant plastics, wherein plastics are preferred for reasons of cost and weight. Especially preferred is polyethylene terephthalate.

### Coloring of the phases

Fortunately, both of the liquid phases can be colored with one or two different colorants of different types. The resulting compound color that is present during the shaking before use is a visual indicator of a mixture that is sufficient for use. In this process, water-insoluble, oil-soluble colorants are used in the hydrophobic phase, and oil-insoluble, water-soluble, or colorants soluble in water/alcohol mixtures are used in the hydrophilic phase.

### Additional additives

The composition according to the present invention can further contain any additive components that are conventional for hair treatment agents, for example, preservatives, solubilizing agents, perfume oils, scents, thickeners, pH buffering agents, conditioning agents such as, for example, plant and herb extracts, protein and silk hydrolysates, photo-protective agents, antioxidants, radical scavengers, anti-dandruff ingredients, shine enhancers, vitamins, softeners, agents to improve combing, etc.

To reduce the heavying effect, particularly with fine hair, the composition according to the present invention is preferably free of salts and oil or fatty type substances, particularly nonvolatile substances, or it contains these substances in very small quantities, e.g. less than 0.5 or less than 0.05 wt%.

An especially preferred embodiment is a product for which the material of the aerosol packaging is made of polyethylene terephthalate, and the composition contains
(A) 50 to 75 wt% water;
(B) 20 to 30 wt% ethanol;
(C) 0.5 to 10 wt% of at least one cationic, nonionic, amphoteric, or zwitterionic polymer, selected from hair-conditioning polymers, hair-setting polymers, and film-forming polymers;
(D) 0.05 to 5 wt% of at least one hair-conditioning cationic surfactant;
(E) 0.1 to 5 wt% of at least one foam-forming or foam-stabilizing surfactant, selected from alkylpolyglucosides with an HLB value of at least 10 and zwitterionic surfactants of the general formula

   R¹-N⁽⁺⁾(R²)₂-A-Z⁽⁻⁾

   wherein R1 represents a linear or branched alkyl, alkenyl, or hydroxyalkyl group with from 8 to 22 C atoms and from 0 to 10 ethylene oxide units and from 0 to I glycerol units or a fatty acid amidoalkylene group with from 8 to 22 C atoms in the fatty acid group and from 1 to 4, preferably 2 or 3, C atoms in the alkylene group; R2 is an alkyl, hydroxyalkyl, or carboxyalkyl group with from 1 to 4 C atoms; A is an alkylene or hydroxyalkylene group with from 1 to 4 C atoms and 0 or 1 hydroxy groups, and Z represents a carboxylate, sulfate, phosphonate, or phosphate group;
wherein the quantities are based on the composition without aerosol propellant, and the composition is filled with aerosol propellants, selected from propane, butane, and mixtures thereof, and wherein the quantity ratio of propellant-free composition to aerosol propellant is in the range of 80:20 to 97:3, and the pressure in the aerosol packaging is a maximum of 3 bar at 20 °C (68 °F).

The product according to the present invention is manufactured by firstly dissolving the components soluble in the hydrophilic phase in the hydrophilic solvent. Before the aerosol propellant is filled, the components that are soluble in the hydrophobic phase are added. Finally, the aerosol propellant is filled, and the aerosol packaging is pressure sealed.

The product can be used as a rinse or as a leave-in. The cosmetic agent according to the present invention is used in that it is applied to the washed hair as a foam and distributed in a quantity sufficient to achieve the desired hair styling effect depending on the amount of hair and the condition of the hair (typically about 3 - 10 g (0.11 - 0.35 oz)). After an action period (e.g. 3 - 6 minutes), it can be rinsed out. The agent can also stay in the hair and the hair is then dried. The object of the invention is thus also a method for hair treatment, wherein
- a foam is created using an aforementioned product according to the present invention;
- this foam is either applied to freshly washed, damp hair, or to dry hair and distributed throughout the hair;
- the composition applied to the hair is left in the hair or rinsed out with water after an action time and
- then the desired hairstyle is produced.

The product according to the present invention can particularly be used to create volume and/or hold for the human hairstyle.

The following examples should serve to illustrate further the object of the present invention.

### Examples

The compositions named below are filled into pressure-resistant containers made of transparent polyethylene terephthalate and then filled with propellant (propane/butane 2.7 bar) in a ratio of 9:1. The containers have foaming heads and are sealed.

### Example 1

| | 1A | 1B | 1C | 1D |
|---|---|---|---|---|
| Polyquatemium-4 (Celquat^{®} L 200) | 2 | 2 | 2 | 2 |
| VinylpyrrolidoneNinyl Acetate Copolymer (Luviskol^{®} VA64) | 2 | 2 | 2 | 2 |
| Coco-Glucoside (53%) | 1 | 1 | 1 | 1 |
| Cetyltrimethyl ammonium chloride | 0.2 | 0.2 | 0.2 | 0.2 |
| Ext. D&C Violet No. 2 (0.1%) | 0.4 | 0.4 | 0.4 | 0.4 |
| FD&C Blue No. 1 (0.1%) | 0.06 | 0.06 | 0.06 | 0.06 |
| Perfume | 0.2 | 0.2 | 0.2 | 0.2 |
| Caffeine | - | - | - | 0.5 |
| Citric acid balance to pH 5-5.5 | q.s. | q.s. | q.s. | q.s. |
| Glycerin (86%) | 5 | 5 | 5 | 5 |
| Ethanol | 20 | 25 | 30 | 25 |
| Water | balance to 100 | balance to 100 | balance to 100 | balance to 100 |

### Example 2

| | 2A | 2B |
|---|---|---|
| Polyvinylpyrrolidone (Luviskol^{®} K90) | 1 | 1 |
| Polyvinylpyrrolidone (Luviskol^{®} K30) | 1 | 1 |
| Chitosan | 0.7 | 0.7 |
| Formic acid (85%) (pH about 4.7-4.9) | 0.21 | 0.21 |
| Coco-Glucoside (53%) | - | 1 |
| Cocamidopropyl Hydroxysultaine | 0.5 | - |
| Cetyltrimethyl ammonium chloride | 0.2 | 0.2 |
| Ext. D&C Violet No. 2 (0.1%) | 0.4 | 0.4 |
| FD&C Blue No. 1 (0.1 %) | 0.06 | 0.06 |
| Perfume | 0.2 | 0.2 |
| Glycerin (86%) | 5 | 5 |
| Ethanol | 25 | 30 |
| Water | balance to 100 | balance to 100 |

### Example 3

| | 3A | 3B | 3C |
|---|---|---|---|
| (Luviskol^{®} K85 CQ solution (polyvinylpyrrolidone, 20%) | 7 | 7 | 7 |
| Vinylpyrrolidone/Vinyl Acetate Copolymer (Luviskol^{®} VA64) | 1 | 1 | 1 |
| Chitosan | 0.95 | 0.9 | 0.95 |
| Luviquat^{®} FC 905 (Polyquaternium-16, 40%) | 0.75 | 0.75 | 0.75 |
| Luviset Clear^{® 1)} | - | 1 | 1 |
| Coco-Glucoside (53%) | 1 | 1 | 1 |
| Decyl Glucoside (55%) | 0.4 | 0.4 | 0.4 |
| Cetyltrimethyl ammonium chloride | 0.2 | 0.2 | 0.2 |
| Formic acid 85% (pH 4.5-4.7) | 0.27 | 0.27 | 0.27 |
| Pyrrolidone carboxylic acid | 0.04 | - | 0.04 |
| Ext. D&C Violet No. 2 (0.1%) | 0.4 | 0.4 | 0.4 |
| FD&C Blue No. 1 (0.1%) | 0.06 | 0.06 | 0.06 |
| Perfume | 0.2 | 0.2 | 0.2 |
| Ethanol | 25 | 25 | 25 |
| Water | balance to 100 | balance to 100 | balance to 100 |

| | | | |
|---|---|---|---|
| ¹⁾ Vinylpyrrolidone/methacrylamide/vinylimidazole copolymer | | | |

### Example 4

| | |
|---|---|
| (Luviskol^{®} K85 CQ solution (polyvinylpyrrolidone, 20%) | 7 |
| Vinylpyrrolidone/Vinyl Acetate Copolymer (Luviskol^{®} VA64) | 1 |
| Chitosan | 1 |
| Luviquat^{®} FC 905 (Polyquaternium-16, 40%) | 0.75 |
| Genagen^{®} KB (Coco-Betaine, 30%) | 1.76 |
| Decyl Glucoside (55%) | 0.2 |
| Cetyltrimethyl ammonium chloride | 0.1 |
| Formic acid (85%) | 0.21 |
| Pyrrolidone carboxylic acid | 0.23 |
| Ext. D&C Violet No. 2 (0.1%) | 0.4 |
| FD&C Blue No. 1 (0.1 %) | 0.06 |
| Perfume | 0.2 |
| Ethanol | 20 |
| Water | balance to 100 |

With all the examples, a soft, microporous foam forms with each release that remains compact for at least about 2 minutes. Both in a non-shaken condition as well as 24 hours after shaking, a clear phase separation of two liquid phases forms.

### Example 5

| | |
|---|---|
| (Luviskol® K85 CQ solution (polyvinylpyrrolidone, 20%) | 7 |
| Vinylpyrrolidone/Vinyl Acetate Copolymer (Luviskol^{®} VA64) | 1 |
| Chitosan | 0.7 |
| Luviquat^{®} FC 905 (Polyquaternium-16, 40%) | 0.75 |
| Cocamidopropyl Betaine | 0.3 |
| Cetyltrimethyl ammonium chloride | 0.1 |
| Formic acid (85%) | 0.21 |
| Pyrrolidone carboxylic acid | 0.23 |
| Ext. D&C Violet No. 2 (0.1%) | 0.4 |
| FD&C Blue No. 1 (0.1%) | 0.06 |
| Perfume | 0.2 |
| Ethanol | 30 |
| Water | balance to 100 |

A nice, microporous foam forms in this process. Both in a non-shaken condition as well as 24 hours after shaking, a phase separation of two separated clear solutions forms.

## Claims

1. Hair care product consisting of transparent, pressure-resistant aerosol packaging, a device for foaming a composition contained in the aerosol packaging and a foaming composition of at least two clear liquid phases separated from each other, wherein the composition contains
(A) water;
(B) at least 15 wt%, based on the composition without propellant, of a water-soluble liquid alcohol;
(C) at least one polymer selected from the hair-conditioning polymers, the hair-setting polymers, and the film-forming polymers;
(D) at least one hair-conditioning cationic surfactant;
(E) at least one foam-forming or foam-stabilizing surfactant, selected from the group consisting of nonionic surfactants, with an HLB value of at least 10 and zwitterionic surfactants;
(F) at least one water-insoluble propellant that is liquified under the pressure conditions in the aerosol packaging.

2. Product as recited in Claim 1, wherein the water-soluble, liquid alcohols (B) are contained in a quantity of from 20 to 35 wt%, based on the composition without aerosol propellant, and selected from monovalent C1 to C4 alcohols and polyvalent C2 to C5 alcohols.

3. Product as recited in one of the previous claims, wherein the foam-forming or foam-stabilizing surfactants (E) are contained in a quantity of from 0.1 to 5 wt%, based on the composition without aerosol propellant, and selected from alkylpolyglucosides and zwitterionic surfactants with betaine or sultaine structures.

4. Product as recited in one of the previous claims, wherein the cationic surfactant (D) is contained in a quantity of from 0.01 to 10 wt%, based on the composition without aerosol propellant, and selected from compounds of the general formula
N⁽⁺⁾R¹R²R³R⁴ X⁽⁻⁾
wherein R1 to R4, independently from one another, mean aliphatic groups, aromatic groups, alkoxy groups, polyoxyalkylene groups, alkylamido groups, hydroxyalkyl groups, aryl groups, or alkaryl groups with 1 to 22 C atoms, wherein at least one of the residues R1 to R4 possesses at least 8 C atoms, and wherein X⁽⁻⁾ represents an anion.

5. Product as recited in one of the previous claims, wherein the polymer (C) is contained in a quantity of from 0.01 to 20 percent by weight and is selected from
- polymers with anionic or anionizable groups, selected from among terpolymers from acrylic acid, ethyl acrylate, and N-tert-butylacrylamide; crosslinked or uncrosslinked vinyl acetate/crotonic acid copolymers; terpolymers from tert-butylacrylate, ethyl acrylate and methacrylic acid; sodium polystyrenesulfonate; copolymers from vinyl acetate, crotonic acid and vinyl propionate; copolymers from vinyl acetate, crotonic acid and vinyl neodecanoate; aminomethylpropanol/acrylate copolymers; copolymers from vinylpyrrolidone and at least one further monomer selected from among acrylic acid, methacrylic acid, acrylic acid esters and methacrylic acid esters; copolymers from methyl vinyl ether and maleic acid monoalkyl esters; aminomethylpropanol salts of copolymers from allyl methacrylate and at least one further monomer selected from among acrylic acid, methacrylic acid, acrylic acid esters and methacrylic acid esters; crosslinked copolymers from ethyl acrylate and methacrylic acid; copolymers from vinyl acetate, mono-n-butyl maleate and isobomyl acrylate; copolymers from two or more monomers selected from among acrylic acid, methacrylic acid, acrylic acid esters and methacrylic acid esters, copolymers from octylacrylamide and at least one monomer selected from among acrylic acid, methacrylic acid, acrylic acid esters and methacrylic acid esters; polyesters from diglycol, cyclohexanedimethanol, isophthalic acid and sulfoisophthalic acid;
- polymers with cationic or cationizable groups, selected from among cationic cellulose derivatives from hydroxyethylcellulose and diallyldimethylammonium chloride; cationic cellulose derivatives from hydroxyethylcellulose and with trimethylammonium substituted epoxides; poly(dimethyldiallylammonium chloride); copolymers from acrylamide and dimethyldiallylammonium chloride; quaternary ammonium polymers, formed from the reaction of diethyl sulfate with a copolymer from vinylpyrrolidone and dimethylaminoethyl methacrylate; quaternary ammonium polymers from methylvinylimidazolium chloride and vinylpyrrolidone; Polyquatemium-35; polymers from trimethylammoniumethyl methacrylate chloride; Polyquatemium-57; dimethylpolysiloxanes substituted with quaternary ammonium groups at the terminal positions; copolymers from vinylpyrrolidone, dimethylaminopropyl methacrylamide and methacryloylaminopropyllauryldimethylammonium chloride; chitosan and its salts; hydroxyalkyl chitosans and their salts; alkylhydroxyalkylchitosans and their salts; N-hydroxyalkylchitosan alkyl ethers; N-hydroxyalkylchitosan benzyl ethers; copolymers from vinylcaprolactam, vinylpyrrolidone and dimethylaminoethyl methacrylate; copolymers from vinylpyrrolidone and dimethylaminoethyl methacrylate, copolymers from vinylpyrrolidone, vinylcaprolactam and dimethylaminopropyl acrylamide; poly- or oligoesters formed from at least one first type of monomer that is selected from among hydroxyacids that are substituted with at least one quaternary ammonium group; terpolymers from vinylpyrrolidone, methacrylamide and vinylimidazole;
- zwitterionic and/or amphoteric polymers, selected from among copolymers from octyl acrylamide, acrylic acid, butylaminoethyl methacrylate, methyl methacrylate and hydroxypropyl methacrylate; copolymers from lauryl acrylate, stearyl acrylate, ethylamine oxide methacrylate and at least one monomer selected from among acrylic acid, methacrylic acid, acrylic acid esters and methacrylic acid esters; copolymers from methacryloyl ethyl betaine and at least one monomer selected from among methacrylic acid and methacrylic acid esters; copolymer from acrylic acid, methyl acrylate and methacrylamidopropyltrimethylammonium chloride; oligomers or polymers that can be prepared from quaternary crotonoylbetaines or quaternary crotonoylbetaine esters;
- nonionic polymers, selected from among polyvinylpyrrolidone, polyvinylcaprolactam, vinylpyrrolidone/vinyl acetate copolymers, polyvinyl alcohol, isobutylene/ethyl maleimide/hydroxyethyl maleimide copolymer; copolymers from vinylpyrrolidone, vinyl acetate and vinylpropionate.

6. Product as recited in one of the previous claims, wherein the polymer (C) is selected from cationic cellulose derivatives of hydroxyethyl cellulose and diallyldimethylammonium chloride; vinylpyrrolidone/vinylacetate copolymers; chitosan; polyvinylpyrrolidone; quaternary ammonium polymers of methylvinylimidazolium chloride and vinylpyrrolidone and combinations of these polymers.

7. Product as recited in one of the previous claims, wherein aerosol propellant (F) is selected from C3 to C4 hydrocarbons, mixtures thereof, as well as mixtures of one or more C3 to C4 hydrocarbons with dimethylether.

8. Product as recited in one of the previous claims, wherein the quantity of aerosol propellant (F) is selected so that the quantity ratio of propellant-free composition to aerosol propellant is in the range of from 80:20 to 97:3, and the pressure in the aerosol packaging is a maximum of 3 bar at 20 °C (68 °F).

9. Product as recited in one of the previous claims, wherein both liquid phases are differently colored using colorants.

10. Product as recited in one of the previous claims, wherein the material of the aerosol packaging is made of polyethylene terephthalate.

11. Product as recited in one of the previous claims, wherein the material of the aerosol packaging is made of polyethylene terephthalate and the composition contains
(A) 50 to 75 wt% water;
(B) 20 to 30 wt% ethanol;
(C) 0.5 to 10 wt% of at least one cationic, nonionic, amphoteric, or zwitterionic polymer, selected from hair-conditioning polymers, hair-setting polymers, and film-forming polymers;
(D) 0.05 to 5 wt% of at least one hair-conditioning cationic surfactant;
(E) 0.1 to 5 wt% of at least one foam-forming or foam-stabilizing surfactant, selected from alkylpolyglucosides with an HLB value of at least 10 and zwitterionic surfactants of the general formula R¹-N⁽⁺⁾(R²)₂-A-Z⁽⁻⁾, wherein R¹ represents a linear or branched alkyl, alkenyl, or hydroxyalkyl group with 8 to 22 C atoms and 0 to 10 ethylene oxide units and 0 to 1 glycerin units or a fatty acid amido alkylene group with from 8 to 22 C atoms in the fatty acid group and 1 to 4 C atoms in the alkylene group; R² is an alkyl, hydroxyalkyl or carboxyalkyl group with from 1 to 4 C atoms; A represents an alkylene or hydroxyalkylene group with from 1 to 4 C atoms and 0 or 1 hydroxy groups, and Z represents a carboxylate, sulfate, sulfonate, phosphonate, or phosphate group;
wherein the quantities are based on the composition without aerosol propellant, and the composition is filled with aerosol propellants, selected from propane, butane, and mixtures thereof, and wherein the quantity ratio of propellant-free composition to aerosol propellant is in the range of 80:20 to 97:3, and the pressure in the aerosol packaging is a maximum of 3 bar at 20 °C (68 °F).

12. A method for hair treatment, wherein
- a foam is created using a product as recited in one of the previous claims;
- this foam is either applied to freshly washed, damp hair, or to dry hair and distributed throughout the hair;
- the composition applied to the hair is left in the hair or rinsed out with water after an action time and
- then the desired hairstyle is produced.

13. Use of a product as recited in one of Claims 1 to 11 to create volume and/or hold in a human hairstyle.

## Patentansprüche

1. Haarpflegeprodukt, das aus einer transparenten, druckbeständigen Aerosolpackung, einer Vorrichtung zum Aufschäumen einer Zusammensetzung, die in der Aerosolpackung enthalten ist, und einer schäumenden Zusammensetzung aus mindestens zwei klaren flüssigen Phasen, die voneinander getrennt sind, besteht, wobei die Zusammensetzung Folgendes enthält:
(A) Wasser;
(B) bezogen auf die Zusammensetzung ohne Treibmittel, mindestens 15 Gew.-% wasserlöslichen flüssigen Alkohol;
(C) mindestens ein Polymer, das ausgewählt ist aus den Haarkonditionierungs-Polymeren, den Haarfestigungs-Polymeren und den filmbildenden Polymeren;
(D) mindestens ein kationisches Haarkonditionierungs-Tensid;
(E) mindestens ein schaumbildendes oder schaumstabilisierendes Tensid, das ausgewählt ist aus der Gruppe bestehend aus nicht-ionischen Tensiden mit einem HLB-Wert von mindestens 10 und zwitterionischen Tensiden;
(F) mindestens ein wasserunlösliches Treibmittel, das sich unter den Druckbedingungen in der Aerosolpackung verflüssigt.

2. Produkt nach Anspruch 1, wobei die wasserlöslichen, flüssigen Alkohole (B), bezogen auf die Zusammensetzung ohne Aerosol-Treibmittel, in einer Menge von 20 bis 35 Gew.-% enthalten sind und ausgewählt sind aus einwertigen C1- bis C4-Alkoholen und mehrwertigen C2- bis C5-Alkoholen.

3. Produkt nach einem der vorangehenden Ansprüche, wobei die schaumbildenden oder schaumstabilisierenden Tenside (E), bezogen auf die Zusammensetzung ohne Aerosol-Treibmittel, in einer Menge von 0,1 bis 5 Gew.-% enthalten sind und ausgewählt sind aus Alkylpolyglucosiden und zwitterionischen Tensiden mit Betain- oder Sultainstrukturen.

4. Produkt nach einem der vorangehenden Ansprüche, wobei das kationische Tensid (D), bezogen auf die Zusammensetzung ohne Aerosol-Treibmittel, in einer Menge von 0,01 bis 10 Gew.-% enthalten ist und ausgewählt ist aus Verbindungen der allgemeinen Formel
N⁽⁺⁾R¹R²R³R⁴ X⁽⁻⁾
wobei R1 bis R4 unabhängig voneinander für aliphatische Gruppen, aromatische Gruppen, Alkoxygruppen, Polyoxyalkylengruppen, Alkylamidgruppen, Hydroxyalkylgruppen, Arylgruppen oder Alkarylgruppen mit 1 bis 22 C-Atomen stehen, wobei mindestens einer der Reste R1 bis R4 mindestens 8 C-Atome besitzt, und wobei X⁽⁻⁾ ein Anion darstellt.

5. Produkt nach einem der vorangehenden Ansprüche, wobei das Polymer (C) in einer Menge von 0,01 bis 20 Gew.-% enthalten ist und ausgewählt ist aus
- Polymeren mit anionischen oder anionisierbaren Gruppen, die ausgewählt sind aus Terpolymeren von Acrylsäure, Ethylacrylat und N-tert-Butylacrylamid; vernetzten oder unvernetzten Vinylacetat/Crotonsäure-Copolymeren; Terpolymeren von tert-Butylacrylat, Ethylacrylat und Methacrylsäure; Natriumpolystyrolsulfonat; Copolymeren von Vinylacetat, Crotonsäure und Vinylpropionat; Copolymeren von Vinylacetat, Crotonsäure und Vinylneodecanoat; Aminomethylpropanol/Acrylat-Copolymeren; Copolymeren von Vinylpyrrolidon und mindestens einem weiteren Monomer, das ausgewählt ist aus Arylsäure, Methacrylsäure, Acrylsäureestern und Methacrylsäureestern; Copolymeren von Methylvinylether und Maleinsäuremonoalkylestern; Aminomethylpropanolsalzen von Copolymeren von Allylmethacrylat und mindestens einem weiteren Monomer, das ausgewählt ist aus Acrylsäure, Methacrylsäure, Acrylsäureestern und Methacrylsäureestern; vernetzten Copolymeren von Ethylacrylat und Methacrylsäure; Copolymeren von Vinylacetat, Mono-n-butylmaleat und Isobornylacrylat; Copolymeren von zwei oder mehreren Monomeren, die ausgewählt sind aus Acrylsäure, Methacrylsäure, Acrylsäureestern und Methacrylsäureestern, Copolymeren von Octylacrylamid und mindestens einem Monomer, das ausgewählt ist aus Acrylsäure, Methacrylsäure, Acrylsäureestern und Methacrylsäureestern; Polyestern von Diglycol, Cyclohexandimethanol, Isophthalsäure und Sulfoisophthalsäure;
- Polymeren mit kationischen oder kationisierbaren Gruppen, ausgewählt aus kationischen Cellulosederivaten von Hydroxyethylcellulose und Diallyldimethylammoniumchlorid; kationischen Cellulosederivaten von Hydroxyethylcellulose und mit Trimethylammonium substituierten Epoxiden; Poly(dimethyldiallylammoniumchlorid); Copolymeren von Acrylamid und Dimethyldiallylammoniumchlorid; quartären Ammoniumpolymeren, gebildet aus der Umsetzung von Diethylsulfat mit einem Copolymer von Vinylpyrrolidon und Dimethylaminoethylmethacrylat; quartären Ammoniumpolymeren von Methylvinylimidazoliumchlorid und Vinylpyrrolidon; Polyquaternium-35; Polymeren von Trimethylammoniumethylmethacrylatchlorid; Polyquaternium-57; Dimethylpolysiloxanen, die an ihren terminalen Positionen mit quartären Ammoniumgruppen substituiert sind; Copolymeren von Vinylpyrrolidon, Dimethylaminopropylmethacrylamid und Methacryloylamino-Propyllauryldimethylammoniumchlorid; Chitosan und dessen Salzen; Hydroxyalkylchitosanen und ihren Salzen; Alkylhydroxyalkylchitosanen und deren Salzen; N-Hydroxyalkylchitosanalkylethern; N-Hydroxyalkylchitosanbenzylethern; Copolymeren von Vinylcaprolactam, Vinylpyrrolidon und Dimethylaminoethylmethacrylat; Copolymeren von Vinylpyrrolidon und Dimethylaminoethylmethacrylat, Copolymeren von Vinylpyrrolidon, Vinylcaprolactam und Dimethylaminopropylacrylamid; Poly- oder Oligoestern, die aus mindestens einer ersten Art Monomer, die ausgewählt ist aus Hydroxysäuren, die mit mindestens einer quartären Ammoniumgruppe substituiert sind, gebildet sind; Terpolymeren von Vinylpyrrolidon, Methacrylamid und Vinylimidazol;
- zwitterionischen und/oder amphoteren Polymeren, die ausgewählt sind aus Copolymeren von Octylacrylamid, Acrylsäure, Butylaminoethylmethacrylat, Methylmethacrylat und Hydroxypropylmethacrylat; Copolymeren von Laurylacrylat, Stearylacrylat, Ethylaminoxidmethacrylat und mindestens einem Monomer, das ausgewählt ist aus Acrylsäure, Methacrylsäure, Acrylsäureestern und Methacrylsäureestern; Copolymeren von Methacryloylethylbetain und mindestens einem Monomer, das ausgewählt ist aus Methacrylsäure und Methacrylsäureestern; Copolymer von Acrylsäure, Methylacrylat und Methacrylamidopropyltrimethylammoniumchlorid; Oligomeren oder Polymeren, die aus quartären Crotonoylbetainen oder quartären Crotonoylbetainestern hergestellt werden können;
- nicht-ionischen Polymeren, die ausgewählt sind aus Polyvinylpyrrolidon, Polyvinylcaprolactam, Vinylpyrrolidon/Vinylacetat-Copolymeren, Polyvinylalkohol, Isobutylen/Ethylmaleimid/Hydroxyethylmaleimid-Copolymer; Copolymeren von Vinylpyrrolidon, Vinylacetat und Vinylpropionat.

6. Produkt nach einem der vorangehenden Ansprüche, wobei das Polymer (C) ausgewählt ist aus kationischen Cellulosederivaten von Hydroxyethylcellulose und Diallyldimethylammoniumchlorid; Vinylpyrrolidon/Vinylacetat-Copolymeren; Chitosan; Polyvinylpyrrolidon; quartären Ammoniumpolymeren von Methylvinylimidazoliumchlorid und Vinylpyrrolidon und Kombinationen dieser Polymere.

7. Produkt nach einem der vorangehenden Ansprüche, wobei das Aerosol-Treibmittel (F) ausgewählt ist aus C3- bis C4-Kohlenwasserstoffen, Mischungen davon, ebenso wie Mischungen aus einem oder mehreren C3- bis C3-Kohlenwasserstoffen mit Dimethylether.

8. Produkt nach einem der vorangehenden Ansprüche, wobei die Menge an Aerosol-Treibmittel (F) so ausgewählt ist, dass das Mengenverhältnis von treibmittelfreier Zusammensetzung zu Aerosol-Treibmittel im Bereich von 80:20 bis 97:3 liegt und der Druck der Aerosolpackung bei 20 °C (68 °F) höchstens 0,3 MPa (3 bar) beträgt.

9. Produkt nach einem der vorangehenden Ansprüche, wobei beide flüssigen Phasen unter Verwendung von Färbemitteln unterschiedlich gefärbt sind.

10. Produkt nach einem der vorangehenden Ansprüche, wobei das Material der Aerosolpackung aus Polyethylenterephthalat besteht.

11. Produkt nach einem der vorangehenden Ansprüche, wobei das Material der Aerosolpackung aus Polyethylenterephthalat besteht und die Zusammensetzung Folgendes enthält:
(A) 50 bis 75 Gew.-% Wasser;
(B) 20 bis 30 Gew.-% Ethanol;
(C) 0,5 bis 10 Gew.-% mindestens eines kationischen, nicht-ionischen, amphoteren oder zwitterionischen Polymers, ausgewählt aus Haarkonditionierungs-Polymeren, Haarfestigungs-Polymeren und filmbildenden Polymeren;
(D) 0,05 bis 5 Gew.-% mindestens eines kationischen Haarkonditionierungs-Tensids;
(E) 0,1 bis 5 Gew.-% mindestens eines schaumbildenden oder schaumstabilisierenden Tensids, ausgewählt aus Alkylpolyglucosiden mit einem HLB-Wert von mindestens 10 und zwitterionischen Tensiden der allgemeinen Formel R¹-N⁽⁺⁾(R²)₂-A-Z⁽⁻⁾, wobei R¹ eine lineare oder verzweigte Alkyl-, Alkenyl- oder Hydroxyalkylgruppe mit 8 bis 22 C-Atomen und 0 bis 10 Ethylenoxideinheiten und 0 bis 1 Glycerineinheiten oder eine Fettsäureamidoethylengruppe mit 8 bis 22 C-Atomen in der Fettsäuregruppe und 1 bis 4 C-Atomen in der Alkylengruppe darstellt; R² eine Alkyl-, Hydroxyalkyl- oder Carboxyalkylgruppe mit 1 bis 4 C-Atomen ist; A eine Alkylen- oder Hydroxyalkylengruppe mit 1 bis 4 C-Atomen und 0 oder 1 Hydroxygruppe darstellt, und Z eine Carboxylat-, Sulfat-, Sulfonat-, Phosphonat- oder Phosphatgruppe darstellt;
wobei die Mengen sich auf die Zusammensetzung ohne Aerosol-Treibmittel beziehen und die Zusammensetzung mit Aerosol-Treibmitteln gefüllt ist, die ausgewählt sind aus Propan, Butan und Mischungen davon, und wobei das Mengenverhältnis von treibmittelfreier Zusammensetzung zu Aerosol-Treibmittel im Bereich von 80:20 bis 97:3 liegt und der Druck in der Aerosolpackung bei 20 °C (68 °F) höchstens 0,3 MPa (3 bar) beträgt.

12. Verfahren zur Haarbehandlung, wobei
- ein Schaum unter Verwendung eines Produkts nach einem der vorangehenden Ansprüche erzeugt wird;
- dieser Schaum entweder auf frisch gewaschenes, feuchtes Haar oder auf trockenes Haar aufgetragen wird und im Haar verteilt wird;
- die auf das Haar aufgetragene Zusammensetzung im Haar belassen wird oder nach einer Einwirkzeit mit Wasser ausgespült wird und
- dann die gewünschte Frisur hergestellt wird.

13. Verwendung eines Produkts nach einem der Ansprüche 1 bis 11, um Volumen zu erzeugen und/oder eine menschliche Frisur zu festigen.

## Revendications

1. Produit de soin capillaire constitué d'un conditionnement en aérosol transparent, résistant à la pression, d'un dispositif pour faire mousser une composition contenue dans le conditionnement en aérosol et d'une composition moussante d'au moins deux phases liquides limpides séparées l'une de l'autre, où la composition contient
(A) de l'eau ;
(B) au moins 15 % en poids, sur base de la composition sans propulseur, d'un alcool liquide hydrosoluble ;
(C) au moins un polymère choisi parmi les polymères de conditionnement des cheveux, les polymères de fixation des cheveux, et les polymères filmogènes ;
(D) au moins un agent tensioactif cationique de conditionnement des cheveux ;
(E) au moins un agent tensioactif formant de la mousse ou stabilisant la mousse, choisi dans le groupe constitué d'agents tensioactifs non ioniques, avec un rapport hydro-lipophile d'au moins 10 et d'agents tensioactifs zwittérioniques ;
(F) au moins un propulseur insoluble dans l'eau qui est liquéfié dans les conditions de pression du conditionnement en aérosol.

2. Produit selon la revendication 1, dans lequel les alcools liquides hydrosolubles (B) sont contenus en une quantité allant de 20 à 35 % en poids, sur base de la composition sans propulseur aérosol, et choisis parmi les alcools monovalents en C1 à C4 et les alcools polyvalents en C2 à C5.

3. Produit selon l'une des revendications précédentes, dans lequel les agents tensioactifs formant de la mousse ou stabilisant la mousse (E) sont contenus en une quantité allant de 0,1 à 5 % en poids, sur base de la composition sans propulseur aérosol, et choisis parmi les alkylpolyglucosides et les agents tensioactifs zwittérioniques avec des structures de bétaïne ou de sultaïne.

4. Produit selon l'une des revendications précédentes, dans lequel l'agent tensioactif cationique (D) est contenu en une quantité allant de 0,01 à 10 % en poids, sur base de la composition sans propulseur aérosol, et choisi parmi les composés de formule générale
N⁽⁺⁾R¹R²R³R⁴ X⁽⁻⁾
dans laquelle R1 à R4, indépendamment les uns des autres, désignent des groupes aliphatiques, des groupes aromatiques, des groupes alcoxy, des groupes polyoxyalkylène, des groupes alkylamido, des groupes hydroxyalkyle, des groupes aryle, ou des groupes alkaryle avec 1 à 22 atomes de C, dans laquelle au moins l'un parmi les résidus R1 à R4 possède au moins 8 atomes de C, et dans laquelle X⁽⁻⁾ représente un anion.

5. Produit selon l'une des revendications précédentes, dans lequel le polymère (C) est contenu en une quantité allant de 0,01 à 20 pour cent en poids et est choisi parmi
- des polymères avec des groupes anioniques ou pouvant être rendus anioniques, choisis parmi des terpolymères d'acide acrylique, acrylate d'éthyle, et N-tert-butylacrylamide ; des copolymères acétate de vinyle/acide crotonique réticulés ou non réticulés ; des terpolymères de tert-butylacrylate, acrylate d'éthyle et acide méthacrylique ; du polystyrènesulfonate de sodium ; des copolymères d'acétate de vinyle, acide crotonique et propionate de vinyle ; des copolymères d'acétate de vinyle, acide crotonique et néodécanoate ; des copolymères aminométhylpropanol/acrylate ; des copolymères de vinylpyrrolidone et au moins un autre monomère choisi parmi l'acide acrylique, l'acide méthacrylique, des esters d'acide acrylique et des esters d'acide méthacrylique ; des copolymères de méthyl-vinyl éther et d'esters monoalkyliques d'acide maléique ; des sels d'aminométhylpropanol de copolymères de méthacrylate d'allyle et d'au moins un autre monomère choisi parmi l'acide acrylique, l'acide méthacrylique, des esters d'acide acrylique et des esters d'acide méthacrylique ; des copolymères réticulés d'acrylate d'éthyle et d'acide méthacrylique ; des copolymères d'acétate de vinyle, de maléate de mono-n-butyle et d'acrylate d'isobornyle ; des copolymères de deux monomères ou plus choisis parmi l'acide acrylique, l'acide méthacrylique, des esters d'acide acrylique et des esters d'acide méthacrylique, des copolymères d'octylacrylamide et d'au moins un monomère choisi parmi l'acide acrylique, l'acide méthacrylique, les esters d'acide acrylique et les esters d'acide méthacrylique ; des polyesters de diglycol, de cyclohexanediméthanol, d'acide isophtalique et d'acide sulfo-isophtalique ;
- des polymères avec des groupes cationiques ou pouvant être rendus cationiques, choisis parmi les dérivés de cellulose cationique d'hydroxyéthylcellulose et de chlorure de diallyldiméthylammonium ; des dérivés de cellulose cationique d'hydroxyéthylcellulose avec des époxydes à substitution triméthylammonium ; du poly(chlorure de diméthyldiallylammonium) ; des copolymères d'acrylamide et de chlorure de diméthyldiallylammonium ; des polymères d'ammonium quaternaire, formés à partir de la réaction de sulfate de diéthyle avec un copolymère de vinylpyrrolidone et de méthacrylate de diméthylaminoéthyle ; des polymères d'ammonium quaternaire de chlorure de méthylvinylimidazolium et de vinylpyrrolidone ; le Polyquaternium-35 ; des polymères de chlorure de méthacrylate de triméthylammonium-éthyle ; le Polyquaternium-57 ; des diméthylpolysiloxanes substitués avec des groupes ammonium quaternaire aux positions terminales ; des copolymères de vinylpyrrolidone, diméthylaminopropyl methacrylamide et chlorure de méthacryloylaminopropyllauryl-diméthylammonium ; le chitosan et ses sels ; des hydroxyalkyl-chitosans et leurs sels ; des alkylhydroxyalkyl-chitosans et leurs sels ; des alkyléthers de N-hydroxyalkyl-chitosan ; des benzyléthers de N-hydroxyalkyl-chitosan ; des copolymères de vinylcaprolactame, vinylpyrrolidone et méthacrylate de diméthylaminoéthyle ; des copolymères de vinylpyrrolidone et de méthacrylate de diméthylaminoéthyle, des copolymères de vinylpyrrolidone, vinylcaprolactame et diméthylaminopropyl-acrylamide ; des poly- ou oligo-esters formés d'au moins un premier type de monomère qui est choisi parmi les hydroxyacides qui sont substitués avec au moins un groupe ammonium quaternaire ; des terpolymères de vinylpyrrolidone, méthacrylamide et vinylimidazole ;
- des polymères zwittérioniques et/ou amphotères, choisis parmi les copolymères d'octyl-acrylamide, acide acrylique, méthacrylate de butylamino-éthyle, méthacrylate de méthyle et méthacrylate d'hydroxypropyle ; des copolymères d'acrylate de lauryle, acrylate de stéaryle, méthacrylate d'oxyde d'éthylamine et au moins un autre monomère choisi parmi l'acide acrylique, l'acide méthacrylique, des esters d'acide acrylique et des esters d'acide méthacrylique ; des copolymères de méthacryloyl-éthyl-bétaïne et au moins un monomère choisi parmi les esters d'acide méthacrylique et d'acide méthacrylique ; un copolymère d'acide acrylique, acrylate de méthyle et chlorure de méthacrylamidopropyl-triméthylammonium ; des oligomères ou polymères qui peuvent être préparés à partir de crotonoylbétaïnes quaternaire ou d'esters de crotonoylbétaïne quaternaires ;
- des polymères non ioniques, choisis parmi la polyvinylpyrrolidone, le polyvinylcaprolactame, des copolymères vinylpyrrolidone/acétate de vinyle, l'alcool polyvinylique, un copolymère isobutylène/éthyl-maléimide/hydroxyéthyl-maléimide ; des copolymères de vinylpyrrolidone, acétate de vinyle et vinylpropionate.

6. Produit selon l'une des revendications précédentes, dans lequel le polymère (C) est choisi parmi des dérivés de cellulose cationique d'hydroxyéthylcellulose et de chlorure de diallyldiméthylammonium ; des copolymères vinylpyrrolidone/vinylacétate ; du chitosan ; de la polyvinylpyrrolidone , des polymères d'ammonium quaternaire de chlorure de méthylvinylimidazolium et de vinylpyrrolidone et des combinaisons de ces polymères.

7. Produit selon l'une des revendications précédentes, dans lequel le propulseur aérosol (F) est choisi parmi des hydrocarbures en C3 à C4, leurs mélanges, ainsi que des mélanges d'un ou plusieurs hydrocarbures en C3 à C4 avec de l'éther diméthylique.

8. Produit selon l'une des revendications précédentes, dans lequel la quantité de propulseur aérosol (F) est choisie de sorte que le rapport de quantité de composition sans propulseur sur propulseur aérosol est dans l'intervalle allant de 80:20 à 97:3, et la pression dans le conditionnement en aérosol est au maximum de 0,3 MPa (3 bar) à 20 °C (68 °F).

9. Produit selon l'une des revendications précédentes, dans lequel l'une et l'autre phase liquide sont colorées différemment à l'aide de colorants.

10. Produit selon l'une des revendications précédentes, dans lequel le matériau du conditionnement en aérosol est constitué de polyéthylène téréphtalate.

11. Produit selon l'une des revendications précédentes, dans lequel le matériau du conditionnement en aérosol est constitué de polyéthylène téréphtalate et la composition contient
(A) 50 à 75 % en poids d'eau ;
(B) 20 à 30 % en poids d'éthanol ;
(C) 0,5 à 10 % en poids d'au moins un polymère cationique, non ionique, amphotère, ou zwittérionique, choisi parmi les polymères de conditionnement des cheveux, les polymères de fixation des cheveux, et les polymères filmogènes ;
(D) 0,05 à 5 % en poids d'au moins un agent tensioactif cationique de conditionnement des cheveux ;
(E) 0,1 à 5 % en poids d'au moins un agent tensioactif formant de la mousse ou stabilisant la mousse, choisi parmi les alkylpolyglucosides avec un rapport hydro-lipophile d'au moins 10 et les agents tensioactifs zwittérioniques de formule générale R¹-N⁽⁺⁾(R²)₂-A-Z⁽⁻⁾, dans laquelle R¹ représente un groupe alkyle, alcényle, ou hydroxyalkyle linéaire ou ramifié avec 8 à 22 atomes de C et 0 à 10 motifs oxyde d'éthylène et 0 à 1 motif glycérine ou un groupe acide gras amido alkylène avec de 8 à 22 atomes de C dans le groupe acide gras et 1 à 4 atomes de C dans le groupe alkylène ; R² est un groupe alkyle, hydroxyalkyle ou carboxyalkyle avec de 1 à 4 atomes de C ; A représente un groupe alkylène ou hydroxyalkylène avec de 1 à 4 atomes de C et 0 ou 1 groupe hydroxy, et Z représente un groupe carboxylate, sulfate, sulfonate, phosphonate, ou phosphate ;
dans lequel les quantités sont basées sur la composition sans propulseur aérosol, et la composition est remplie avec des propulseurs aérosols, choisis parmi le propane, le butane, et leurs mélanges, et dans lequel le rapport de quantité de composition sans propulseur sur propulseur aérosol est dans l'intervalle de 80:20 à 97:3, et la pression dans le conditionnement en aérosol est un maximum de 0,3 MPa (3 bar) à 20 °C (68 °F).

12. Procédé de traitement capillaire, dans lequel
- une mousse est créée en utilisant un produit selon l'une des revendications précédentes;
- cette mousse est ou appliquée sur des cheveux humides fraîchement lavés, ou sur des cheveux secs et répartie sur l'ensemble des cheveux ;
- la composition appliquée sur les cheveux est laissée dans les cheveux ou rincée avec de l'eau après un temps d'action et
- ensuite la coiffure souhaitée est produite.

13. Utilisation d'un produit selon l'une des revendications 1 à 11, de façon à créer un volume et/ou une tenue dans une coiffure humaine.
